# EUROPEAN PATENT SPECIFICATION

(11) **EP 3 419 627 B1**
(45) Date of publication and mention of the grant of the patent: **12.07.2023**
(21) Application number: 17712031.8
(22) Date of filing: 24.02.2017
(51) Int. Cl.: C07D 201/00, A61K 31/53, C07D 253/10, A61P 31/14

(54) **PROCESS FOR THE MANUFACTURE OF ANTIVIRAL COMPOUNDS, SUCH AS TRIAZAVIRIN**
VERFAHREN ZUR HERSTELLUNG VON ANTIVIRALEN VERBINDUNGEN WIE TRIAZAVIRIN
PROCÉDÉ DE FABRICATION DE COMPOSÉS ANTIVIRAUX, TELS QUE LA TRIAZAVIRINE

(30) Priority: 24.02.2016 EP 16157237
(43) Date of publication of application: 02.01.2019
(73) Proprietor: Doring International GmbH, 8884 Oberterzen (CH)
(72) Inventor: SOROKIN, Pavel Vladimirovich, Novouralsk City 624130 (RU); THORNE, David Edmund, 8884 Oberterzen (CH)
(74) Representative: Gevers Patents
(86) International application number: PCT/EP2017/054422
(87) International publication number: WO 2017/144708

(56) References cited:
- WO-A1-2015/016818
- RU-C1- 2 294 936
- RU-C2- 2 343 154
- RU-C2- 2 404 182
- RU-C2- 2 457 844

## Description

### FIELD OF INVENTION

The present invention relates to methods for manufacturing of 7-thio-substituted-3-nitro-1,2,4-triazolo [5,1-c]-1,2,4-triazin-4(1H)-one compounds.

### BACKGROUND OF THE INVENTION

Negative-sense, single-stranded RNA viruses (ssRNA), such as ssRNA viruses belonging to the Order Mononegavirales such as viruses in the Rhabdoviridae family, in particular the Rabies virus, the Filoviridae family, in particular the Ebolavirus, and the Paramyxoviridae family, in particular the Measles virus, other ssRNA viruses belonging to unassigned families such as notably the Arenaviridae family, the Bunyaviridae family and the Orthomyxoviridae family and other unassigned ssRNA viruses such as notably the Deltavirus, cause many diseases in wildlife, domestic animals and humans. These ssRNA viruses belonging to different families are genetically and antigenically diverse, exhibiting broad tissue tropisms and a wide pathogenic potential.

For example, the Filoviridae viruses belonging to the Order Mononegavirales, in particular the Ebolaviruses and Marburgviruses, are among the most lethal and most destructive viruses in the world. Filoviridae viruses are of particular concern as possible biological weapons since they have the potential for aerosol dissemination and weaponization.

The Ebolavirus includes five species: the Zaire, Sudan, Reston, Taï Forest and Bundibugyo Ebolaviruses. In particular the Zaire, Sudan and Bundibugyo Ebolavirus cause severe, often fatal, viral hemorraghic fevers in humans and nonhuman primates.

For more than 30 years, the Ebolavirus has been associated with periodic episodes of hemorrhagic fever in Central Africa that produce severe disease in infected patients. Mortality rates in outbreaks have ranged from 50% for the Sudan species of the Ebolavirus to up to 90% for the Zaire species of the Ebolavirus ((Sanchez et al., Filoviridae: Marburg and Ebola Viruses, in Fields Virology, pages 1409-1448 (Lippincott Williams & Wilkins, Philadelphia)). In November 2007, during an outbreak in the Bundibugyo district of Uganda, near the border with the Democratic Republic of the Congo the fifth species of the Ebolavirus was discovered, the Bundibugyo species. Said outbreak resulted in a fatality rate of about 25% (Towner et al., PLoS Pathog., 4(11) :e1000212 (2008)). The Zaire species of the Ebolavirus has also decimated populations of wild apes in this same region of Africa (Walsh et al., Nature, 422:611-614 (2003)).

When infected with the Ebolavirus, the onset of illness is abrupt and is characterized by high fever, headaches, joint and muscle aches, sore throat, fatigue, diarrhea, vomiting, and stomach pain. A rash, red eyes, hiccups and internal and external bleeding may be seen in some patients. Within one week of becoming infected with the virus, most patients experience chest pains and multiple organ failure, go into shock, and die. Some patients also experience blindness and extensive bleeding before dying.

Another example of a negative sense single-stranded RNA envelope virus is the Morbilllivirus such as the Measles virus which is associated with Measles and the Lyssavirus such as the Rabies virus.

The Lyssavirus, belonging to the family Rhabdoviridae, includes eleven recognized species, in particular the Rabies virus which is known to cause Rabies. Rabies is an ancient disease with the earliest reports possibly dated to the Old World before 2300 B.C and remains a world health threat due to remaining lack of effective control measures in animal reservoir populations and a widespread lack of human access to vaccination. The Rabies virus is distributed worldwide among mammalian reservoirs including carnivores and bats. Each year there are many reported cases of transmission of the Rabies virus from animals to humans (e.g. by an animal bite). More than 50,000 people annually die of Rabies, particularly in Asia and Africa.

### SUMMARY OF THE INVENTION

One object of the present invention is to provide a process for manufacturing an antiviral compound (A) of general formula (I) or a pharmaceutically acceptable salt thereof 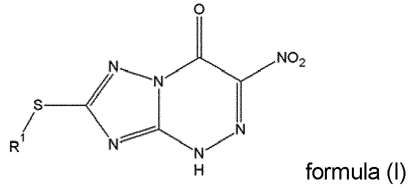 wherein
- R¹ is selected from an alkyl group having 1 to 12 carbon atoms which is optionally substituted by a halogen atom or a hydroxyl group, a cycloalkyl group having 3 to 12 carbon atoms which is optionally substituted by a halogen atom or a hydroxyl group, an aryl group, an alkylaryl group, or a cycloheteroalkyl group having 3 to 12 carbon atoms, preferably an alkyl group having 1 to 8 carbon atoms optionally substituted by a halogen atom or hydroxyl group, a cycloalkyl group having 3 to 6 carbon atoms which is optionally substituted by a halogen atom or hydroxyl group, a phenyl, a benzyl, a morpholine, or an imidazolyl, more preferably an alkyl group having 1 to 4 carbon atoms, a cyclohexyl, a phenyl or a benzyl, and
   which comprises the steps of :
   Step 1. : diazotization of a compound (B) of general formula (II) : 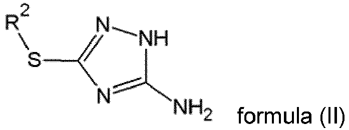 wherein R² is selected from hydrogen, thereby forming a diazonium compound of general formula (III) or a pharmaceutically acceptable salt thereof: 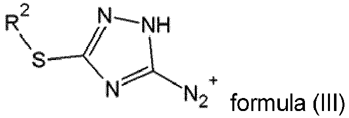 wherein R2 has the same meaning as defined here above,
   Step 2. : condensation reaction of the diazonium compound of general formula (III) or a pharmaceutically acceptable salt thereof, as obtained in Step 1, with at least one α-nitroester of general formula (IV), as shown below, 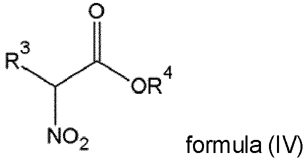 wherein
- R³ is independently selected from a group consisting of:
   - -(CO)-OR⁵ wherein R⁵ is an alkyl group having 1 to 24 carbon atoms which is optionally substituted by a hydroxyl group, an amine group, a halogen atom, an aryl group or an aralkyl group;
   - -(CO)-R⁶ wherein R⁶ is an alkyl group having 1 to 12 carbon atoms which is optionally substituted by a hydroxyl group, an amine group, a halogen atom, an aryl group or an aralkyl group,
- R⁴ is an alkyl group having 1 to 24 carbon atoms which is optionally substituted by a hydroxyl group, an amine group, a halogen atom, an aryl group or an aralkyl group; an alkenyl group.

It is also a further object of the present invention to provide a process for the manufacture of an antiviral compound (A) of general formula (I) or a pharmaceutically acceptable salt thereof 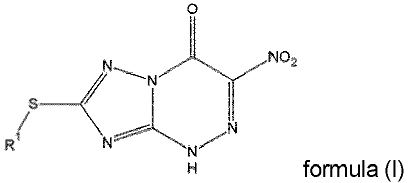 wherein
- R¹ is selected from an alkyl group having 1 to 12 carbon atoms which is optionally substituted by a halogen atom or hydroxyl group, a cycloalkyl group having 3 to 12 carbon atoms which is optionally substituted by a halogen atom or hydroxyl group, an aryl group, an alkylaryl group, or a cycloheteroalkyl group having 3 to 12 carbon atoms, and
   which comprises the steps of:
   Step 1: diazotization of a compound (B) of general formula (II) 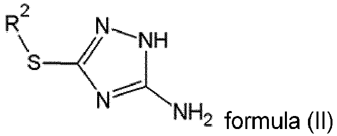 wherein R² is equal to R¹, as defined here above, thereby forming a diazonium compound of general formula (III) or a pharmaceutically acceptable salt thereof: 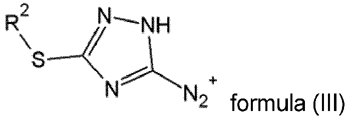 wherein R² has the same meaning as defined here above,
   Step 2. : condensation reaction of the diazonium compound of general formula (III) or a pharmaceutically acceptable salt thereof, as obtained in Step 1, with at least one α-nitroester of general formula (IV), as shown below, 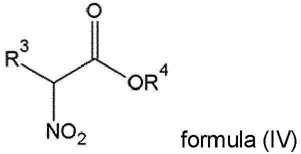 wherein
- R³ is -(CO)-R⁶ is wherein R⁶ is an alkyl group having 1 to 12 carbon atoms which is optionally substituted by a hydroxyl group, an amine group, a halogen atom, an aryl group or an aralkyl group,
- R⁴ is an alkyl group having 1 to 24 carbon atoms which is optionally substituted by a hydroxyl group, an amine group, a halogen atom, an aryl group or an aralkyl group; an alkenyl group.

As used herein the term "alkyl" has the broadest meaning generally understood in the art, and may include a moiety which is linear, branched or a combination thereof.

As used herein the term "cycloalkyl group having 3 to 12 carbon atoms" is a non-aromatic carbon-based ring composed of at least three carbon atoms and at most 12 carbon atoms. Examples of cycloalkyl group having 3 to 12 carbon atoms include, but are not limited to, cyclopropyl, cyclobutyl, cyclopentyl, cyclohexyl, etc.

As used herein the term "aryl group" is any carbon-based aromatic group including, but not limited to, phenyl, tolyl, xylyl, cumenyl, naphthyl, etc. The term "aromatic" also includes "heteroaryl group," which is defined as an aromatic group that has at least one heteroatom incorporated within the ring of the aromatic group. Examples of heteroatoms include, but are not limited to, nitrogen, oxygen, sulfur, and phosphorus. Examples of heteroaryl groups include, but are not limited to, furyl, thienyl, pyrrolyl, imidazolyl, pyridyl, pyrazyl, pyrimidinyl, indolyl, carbazolyl, isoxazolyl, isothiazolyl etc. The aryl group can be substituted or unsubstituted. The aryl group can be substituted with one or more groups including, but not limited to, alkyl, alkynyl, alkenyl, aryl, halide, nitro, amino, ester, ketone, aldehyde, hydroxy, carboxylic acid, or alkoxy.

As used herein the term "alkylaryl group" refers preferably to an alkylaryl group having 6 to 20 carbon atoms such as, but not limited to, benzyl, phenethyl, naphthylmethyl, optionally substituted with one or more groups including, but not limited to, alkyl, alkynyl, alkenyl, aryl, halide, nitro, amino, ester, ketone, aldehyde, hydroxy, carboxylic acid, or alkoxy.

As used herein the term "heterocycloalkyl group having 3 to 12 carbon atoms" is a cycloalkyl group as defined above where at least one of the carbon atoms of the ring is substituted with a heteroatom such as, but not limited to, nitrogen, oxygen, sulphur, or phosphorus. Examples of heterocycloalkyl groups having 3 to 12 carbon atoms include, but are not limited to, morpholine, a piperidine, n-methyl-piperazine etc.

Preferably, R¹ in the antiviral compound (A) or the pharmaceutically acceptable salt thereof, is selected from an alkyl group having 1 to 8 carbon atoms optionally substituted by a halogen atom or hydroxyl group, a cycloalkyl group having 3 to 6 carbon atoms which is optionally substituted by a halogen atom or hydroxyl group, a phenyl, a benzyl, a morpholine, or an imidazolyl. More preferably, R¹ is selected from an alkyl group having 1 to 4 carbon atoms, a cyclohexyl, a phenyl or a benzyl. Even more preferably R¹ is selected from methyl, ethyl, propyl or isopropyl. Most preferably, R¹ is a methyl group.

In a preferred embodiment of the present invention, the antiviral compound (A), as detailed above, is in the form of a pharmaceutically acceptable salt.

It is understood that the antiviral compound (A), as detailed above, possessing a sufficiently acid function generally include the corresponding pharmaceutically acceptable salts of inorganic bases and organic bases.

Therefore, in the present invention the term "pharmaceutically acceptable salts" refers to salts prepared from pharmaceutically acceptable non-toxic bases including inorganic bases and organic bases. Salts derived from nonorganic bases notably include sodium, potassium, lithium, ammonia, calcium, magnesium, ferrous, zinc, manganous, aluminum, ferric, manganic salts and the like, sodium is especially preferred. Salts derived from pharmaceutically acceptable organic non-toxic bases include salts of primary, secondary, tertiary and quaternary amines, substituted amines including naturally occurring substituted amines, cyclic amines and basic ion exchange resins, such as triethylamine, tripropylamine, 2- dimethylaminoethanol, 2-diethylaminoethanol, lysine, arginine, histidine, caffeine, procaine, N-ethylpiperidine, hydrabamine, choline, betaine, ethylenediamine, glucosamine, methylglycamine, theobromine, purines, piperazine, piperidine, polyamine resins and the like, arginine and lysine are especially preferred.

It is further understood that said pharmaceutically acceptable salts of antiviral compound (A) may be in the form of a solvate. Among solvates mention may be notably made of hydrates.

The term "solvate" is intended to refer to a crystal structure incorporating either stoichiometric or non-stoichiometric amounts of solvent. In case the solvate is a hydrate, said solvent is water.

For example, when the pharmaceutically acceptable salts of antiviral compound (A) are produced in a mixture of water and an organic solvent miscible with water, said pharmaceutically acceptable salts of antiviral compound (A) are produced in the form of a hydrate.

Known methods for the manufacturing of antiviral compound (A) of general formula (I) or a pharmaceutically acceptable salt thereof are notably described by V. L. Rusinov et al. in Pharmaceutical Chemistry Journal, September 1990, Volume 24, Issue 9, pp 646-650, Russian Pat. Nos. 2294936 and 2536874.

In these methods, the antiviral compound (A) or the pharmaceutically acceptable salt thereof, is prepared starting from 5-amino-3-methylthio-1,2,4-triazole which is subjected to a diazotization step thereby forming a diazonium salt, followed by an aza-coupling of said diazonium salt with α-nitroesters, in particular ethyl-nitroacetate. The latter compound is known to be obtained only in low yields. It is generally prepared by the nitration of acetoacetic ester with acetyl nitrate in low yield from 27 to 47% (Journal of the Chemical Society, 1958, p.2276-82 and Bulletin de la Societe Chimique de France, 31, 847-854; 1904). Thus, this synthesis suffers from having low yields and the explosiveness of acetyl nitrate.

Known methods to prepare 5-amino-3-methylthio-1,2,4-triazole are notably described by A.V. Dolzhenko et al., in Heterocycles, 2007, volume. 71, No. 2, pages 429-436, compound, and by SM Desenko et al. in Folio:Kharkov, 1998, pages 122 - 123.

Also, RU2343154 discloses a process for preparing the sodium salt of 2-methylthio-6-nitro-1,2,4-triazolo[5,1-c]-1,2,4-triazin-7-one dehydrate consisting in that the salt of 3-methylthio-1,2,4-triazolyl-5-diazonium which is obtained by diazotization of 5-amino-3-methylthio-1,2,4-triazole action with 0.1M sodium nitrite and acid, reacted in a condensation reaction with diethylnitromalonate in the presence of sodium carbonate or sodium hydroxide at 0-25 °C in aqueous-alcoholic medium.

One of the drawbacks of these known methods is the synthesis of the 5-amino-3-methylthio-1,2,4-triazole, the key precursor of 3-methylthio-1,2,4-triazol-5-yl-diazonium. In the method described by A.V. Dolzhenko et al., the synthesis of 5-amino-3-methylthio-1,2,4-triazole involves the use of the toxic carbon disulphide (CS₂) and the formation of an unstable cyanamide intermediate compound. In the method described by SM Desenko, the synthesis of 5-amino-3-methylthio-1,2,4-triazole involves the alkylation of 5-amino-3-mercapto-1,2,4-triazole with methyl iodide. Low yields of the key intermediate in the alkylation step are the disadvantage of this latter method.

In view of the above, there is thus a need for improved methods to manufacture the antiviral compound (A) or the pharmaceutically acceptable salt thereof, as detailed above.

It has now been found new processes for the manufacture of the antiviral compound (A) or the pharmaceutically acceptable salt thereof which allows for improved yield, high efficiency and lower manufacturing cost in particular compared to the known processes whereby 5-amino-3-methylthio-1,2,4-triazole is used as starting material.

The invention consequently relates to a process for the manufacture of the antiviral compound (A) of general formula (I) or a pharmaceutically acceptable salt thereof 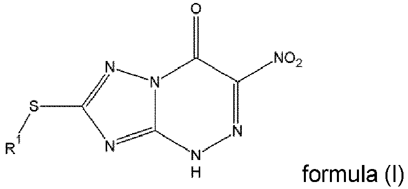 wherein
- R¹ is selected from an alkyl group having 1 to 12 carbon atoms which is optionally substituted by a halogen or hydroxyl group, a cycloalkyl group having 3 to 12 carbon atoms which is optionally substituted by a halogen or a hydroxyl group, an aryl group, an alkylaryl group, or a cycloheteroalkyl group having 3 to 12 carbon atoms,
   which comprises the steps of :
   Step 1. : diazotization of a compound (B) of general formula (II) : 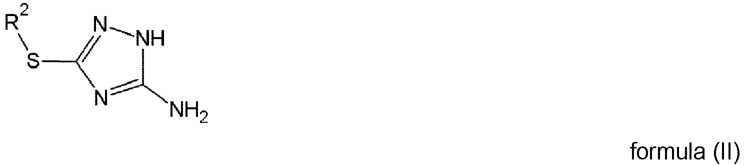 wherein R² is selected from hydrogen, thereby forming a diazonium compound of general formula (III) or a pharmaceutically acceptable salt thereof: 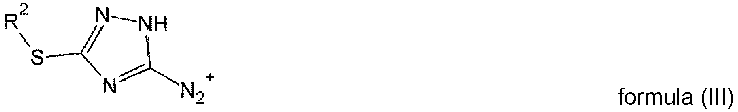 wherein R² has the same meaning as defined here above,
   Step 2. : condensation reaction of the diazonium compound of general formula (III) or a pharmaceutically acceptable salt thereof, as obtained in Step 1, with at least one α-nitroester of general formula (IV), as shown below, 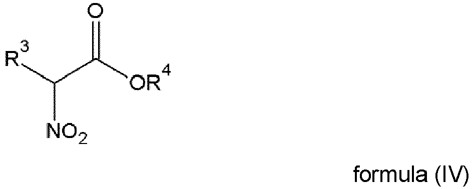 wherein
- R³ is independently selected from a group consisting of:
   - -(CO)-OR⁵ wherein R⁵ is an alkyl group having 1 to 24 carbon atoms which is optionally substituted by a hydroxyl group, an amine group, a halogen atom, an aryl group or an aralkyl group;
   - -(CO)-R⁶ wherein R⁶ is an alkyl group having 1 to 12 carbon atoms which is optionally substituted by a hydroxyl group, an amine group, a halogen atom, an aryl group or an aralkyl group,
- R⁴ is an alkyl group having 1 to 24 carbon atoms which is optionally substituted by a halogen atom, an aryl group or an aralkyl group; an alkenyl group

The invention further relates to a process for the manufacture of an antiviral compound (A) of general formula (I) or a pharmaceutically acceptable salt thereof 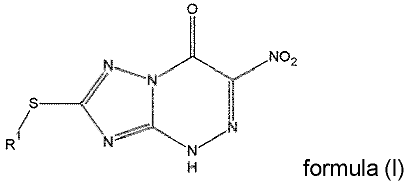 wherein
- R¹ is selected from an alkyl group having 1 to 12 carbon atoms which is optionally substituted by a halogen atom or hydroxyl group, a cycloalkyl group having 3 to 12 carbon atoms which is optionally substituted by a halogen atom or hydroxyl group, an aryl group, an alkylaryl group, or a cycloheteroalkyl group having 3 to 12 carbon atoms, and
   which comprises the steps of:
   Step 1: diazotization of a compound (B) of general formula (II) 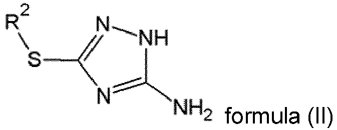 wherein R² is equal to R¹, as defined here above, thereby forming a diazonium compound of general formula (III) or a pharmaceutically acceptable salt thereof: 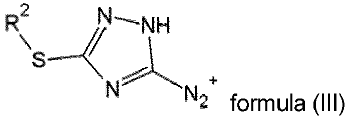 wherein R² has the same meaning as defined here above,
   Step 2. : condensation reaction of the diazonium compound of general formula (III) or a pharmaceutically acceptable salt thereof, as obtained in Step 1, with at least one α-nitroester of general formula (IV), as shown below, 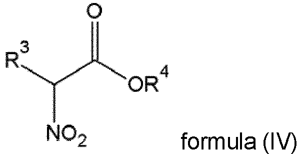 wherein
- R³ is -(CO)-R⁶ is wherein R⁶ is an alkyl group having 1 to 12 carbon atoms which is optionally substituted by a hydroxyl group, an amine group, a halogen atom, an aryl group or an aralkyl group,
- R⁴ is an alkyl group having 1 to 24 carbon atoms which is optionally substituted by a hydroxyl group, an amine group, a halogen atom, an aryl group or an aralkyl group; an alkenyl group.

It is further understood that all definitions and preferences, as described above, equally apply for the process for the manufacture of the antiviral compound (A) of general formula (I) or a pharmaceutically acceptable salt thereof, as detailed above and equally apply for all further embodiments, as described below.

Preferably, R⁴ is an alkyl group having 1 to 12 carbon atoms which is optionally substituted by a hydroxyl group, an amine group, a halogen atom, an aryl group or an aralkyl group, more preferably R⁴ is an alkyl group having 1 to 8 carbon atoms which is optionally substituted by a hydroxyl group, an amine group, a halogen atom, an aryl group or an aralkyl group, more preferably an alkyl group having 1 to 6 carbon atoms, even more preferably an alkyl group having 1 to 4 carbon atoms. Most preferably, R⁴ is chosen among methyl, ethyl, propyl or isopropyl.

Preferably, R⁵ is an alkyl group having 1 to 12 carbon atoms which is optionally substituted by a hydroxyl group, an amine group, a halogen atom, an aryl group or an aralkyl group, more preferably R⁵ is an alkyl group having 1 to 8 carbon atoms which is optionally substituted by a hydroxyl group, an amine group, a halogen atom, an aryl group or an aralkyl group, more preferably an alkyl group having 1 to 6 carbon atoms, even more preferably an alkyl group having 1 to 4 carbon atoms. Most preferably, R⁵ is chosen among methyl, ethyl, propyl or isopropyl.

Preferably, R⁶ is an alkyl group having 1 to 8 carbon atoms which is optionally substituted by a hydroxyl group, an amine group, a halogen atom, an aryl group or an aralkyl group, more preferably R⁶ is an alkyl group having 1 to 6 carbon atoms, even more preferably an alkyl group having 1 to 4 carbon atoms. Most preferably, R⁶ is chosen among methyl, ethyl, propyl or isopropyl.

Preferred α-nitroester of general formula (IV), as detailed above, suitable to be used in the condensation reaction of the the diazonium compound of general formula (III) or a pharmaceutically acceptable salt thereof are dimethyl nitromalonate, diethyl nitromalonate, dipropyl nitromalonate, diisopropyl nitromalonate, dibutyl nitromalonate, diisobutyl nitromalonate, di-sec-butyl nitromalonate, di-tert-butyl nitromalonate, 2-nitro-methylacetoacetate, 2-nitro-ethylacetoacetate, 2-nitro-propylacetoacetate, 2-nitro-isopropylacetoacetate, 2-nitro-butylacetoacetate, 2-nitro-isobutylacetoacetate, 2-nitro-sec-butylacetoacetate, 2-nitro-tert-butylacetoacetate, more preferably dimethyl nitromalonate, diethyl nitromalonate, 2-nitro-methylacetoacetate, 2-nitro-ethylacetoacetate.

According to certain embodiments in the process of the present invention, R² is a hydrogen and the α-nitroester is having the general formula (IV), as shown below, 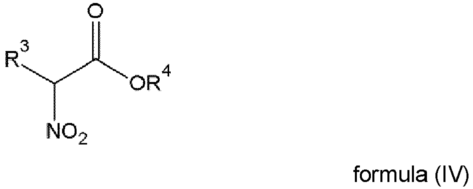 wherein R³ is -(CO)-OR⁵ wherein R⁵ is an alkyl group having 1 to 24 carbon atoms which is optionally substituted by a hydroxyl group, an amine group, a halogen atom, an aryl group or an aralkyl group, preferably R⁵ is an alkyl group having 1 to 12 carbon atoms which is optionally substituted by a hydroxyl group, an amine group,

a halogen atom, an aryl group or an aralkyl group, more preferably R⁵ is an alkyl group having 1 to 8 carbon atoms which is optionally substituted by a hydroxyl group, an amine group, a halogen atom, an aryl group or an aralkyl group, more preferably an alkyl group having 1 to 6 carbon atoms, even more preferably an alkyl group having 1 to 4 carbon atoms. Even more preferably, R⁵ is methyl, ethyl, propyl or isopropyl. Most preferably, R⁵ is ethyl.

According to certain embodiments in the process of the present invention, R² is equal to R¹, as defined here above, and the α-nitroester is having the general formula (IV), as defined above, as shown below, 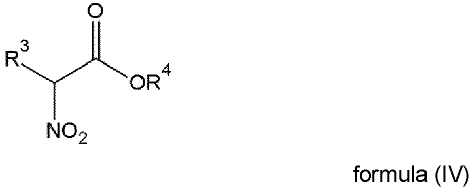 wherein R³ is -(CO)- R⁶ wherein R⁶ is methyl.

The diazotization of the compound (B) of general formula (II), as detailed above, can be performed by means of diazotization methods that are known to a person skilled in the art, preferably by using sodium nitrite (NaNO₂) or nitrosylsulfuric acid (HNOsS) in acidic medium using inorganic acids such as hydrochloric acid, sulfuric acid or phosphoric acid or mixtures thereof or organic acids such as acetic acid, trifluoroacetic acid or propionic acid or mixtures thereof. Also mixtures of inorganic acid with organic acids can be advantageously used.

Generally, the diazotization of the compound (B) of general formula (II), as detailed above, is carried out at a temperature equal to or higher than -25 °C, preferably equal to or higher than -20 °C, more preferably equal to or higher than - 15 °C. It is generally carried out at a temperature equal to or lower than 15 °C, preferably at a temperature equal to or lower than 10 °C, more preferably a temperature equal to or lower than 5 °C. A temperature ranging from -10 °C to 5 °C is most preferred.

If desired, aqueous solutions of sodium nitrite (NaNO₂) or nitrosylsulfuric acid (HNOsS), in particular an aqueous solution of sodium nitrite (NaNO₂) can be used in the form of crushed ice.

It should be mentioned that the use of such a "cold" diazotization method, as described above, advantageously ensures the safety of the process of the present invention thereby preventing an uncontrolled process of foam formation with possible subsequent detonation due to increased pressure in the system, e.g. formation of the foam - the loss of substance - disintegration of nitrogen-containing matter.

In the condensation reaction of the diazonium compound of general formula (III) or a pharmaceutically acceptable salt thereof with at least one α-nitroester of general formula (IV), as detailed above, according to Step 2. of the process of the present invention, the molar ratio of the diazonium compound of general formula (III), as detailed above, to the α-nitroester of general formula (IV), as detailed above, is advantageously from 0.5:2 to 2:0.5, preferably from 0.7:1.5 to 1.5:0.7, and more preferably from 0.8:1.2 to 1.2:0.8, even more preferably 0.9:1 to 1:0.9. Most preferably, the molar ratio is 1:1.

According to one embodiment, the condensation reaction in Step 2. of the diazonium compound of general formula (III) or a pharmaceutically acceptable salt thereof, with the at least one α-nitroester of general formula (IV), as detailed above, is carried out in the presence of at least one pharmaceutically acceptable non-toxic inorganic or organic base.

Typical examples of said pharmaceutically acceptable non-toxic inorganic bases may include alkali metal hydroxides such as sodium hydroxide, potassium hydroxide, lithium hydroxide; alkaline earth metal hydroxides such as calcium hydroxide, magnesium hydroxide; basic alkali metal salts such as sodium carbonate, sodium hydrogencarbonate, potassium carbonate and potassium hydrogencarbonate; ammoniac. Preferred pharmaceutically acceptable non-toxic inorganic bases are sodium hydroxide and potassium hydroxide. Most preferred pharmaceutically acceptable non-toxic inorganic base is sodium hydroxide.

Typical examples of said pharmaceutically acceptable non-toxic organic bases may include primary, secondary, tertiary and quaternary amines, substituted amines including naturally occurring substituted amines, cyclic amines and basic ion exchange resins, such as triethylamine, tripropylamine, 2- dimethylaminoethanol, 2-diethylaminoethanol, lysine, arginine, histidine, caffeine, procaine, N-ethylpiperidine, hydrabamine, choline, betaine, ethylenediamine, glucosamine, methylglycamine, theobromine, purines, piperazine, piperidine, polyamine resins and the like. Preferred pharmaceutically acceptable non-toxic organic bases are arginine and lysine. Most preferred pharmaceutically acceptable non-toxic organic base is arginine.

The amount of said pharmaceutically acceptable non-toxic inorganic or organic bases are selected as such that the reaction mixture reaches a pH of between 6.0 to 12.0, preferably between 7.0 to 11.0, more preferably between 7.5 and 10.5.

The manner of addition of said pharmaceutically acceptable non-toxic inorganic or organic bases in Step 2. is selected as such that the temperature of the reaction mixture remains below 10 °C, preferably below 5 °C.

Generally, the condensation of the diazonium compound of general formula (III), as detailed above, to the at least one α-nitroester of general formula (IV), as detailed above, is carried out at a temperature equal to or lower than 15 °C, preferably equal to or lower than 10 °C, more preferably equal to or lower than 5 °C. It is generally carried out at a temperature equal to or higher than -25 °C, preferably at a temperature equal to or higher than -15 °C, more preferably a temperature equal to or higher than -10 °C. A temperature ranging from -10 °C to 5 °C is most preferred.

If desired, the at least one α-nitroester of general formula (IV), as detailed above, can be used in combination with a solvent selected from an alcohol such as methanol, ethanol, n-propyl alcohol, isopropyl alcohol, tert-butyl alcohol, n-butyl alcohol, iso-butyl alcohol and the like; an ether such as dimethylether, diethylether, dipropylether, diisopropylether, di-tert-butylether, diisobutylether, dibutylether, di-sec-butylether, dioxane, tetrahydrofuran, dimethoxymethane, dimethoxyethane, dimethoxypropane and the like; a ketone such as dimethylketone, diethylketone, dipropylketone, methylethylketone and the like; a sulfur-containing solvent such as dimethylsulfoxide, dimethylsulfone, diphenylsulfone, diethylsulfoxide, diethylsulfone, diisopropylsulfone, tetrahydrothiophene-1, 1-dioxide (commonly called tetramethylene sulfone or sulfolane), tetrahydrothiophene-1-monoxide and the like; a nitrogen-containing polar aprotic solvent such as dimethylacetamide, dimethylformamide and N-methyl pyrrolidinone (i.e., NMP) and the like; and mixtures thereof.

If appropriate, the solvent is generally used in excess. Advantageously the solvent is used in more than a thenfold excess, preferably more than a fifteenfold excess relative to the amount of the at least one α-nitroester of general formula (IV), as detailed above.

If desired, the alcohol can be applied in the form of an aqueous alcoholic solution, an organic solvent or in combination with at least one alkali metal alkoxide.

Typical examples of alkali metal alkoxides notably include but not limited to sodium methoxide, sodium isopropoxide, potassium ethoxide and the like.

For the purpose of the present invention, the term "alcoholic solution" refers to solutions of an alcohol in water, in salt water or in any other aqueous mineral salt solution.

When R² is a hydrogen then 7-mercapto-3-nitro-1,2,4-triazolo[5,1-c]-1,2,4-triazin-4(1H)-one or a pharmaceutically acceptable salt thereof is formed in Step 2. of the process for the manufacture of the antiviral compound (A) of general formula (I) or a pharmaceutically acceptable salt thereof.

According to this embodiment in the process of the present invention, 7-mercapto-3-nitro-1,2,4-triazolo[5,1-c]-1,2,4-triazin-4(1H)-one or a pharmaceutically acceptable salt thereof, as formed in Step 2. is further subjected to an alkylation reaction with an alkylating agent of formula (V): R¹-X or formula (VI) R¹-Z wherein wherein R¹ has the same meaning as defined here above, X is a halogen atom or an oxygen containing functional groups such as OTos and OTMS, and Z is a sulfate group (i.e., O-S(=O)₂-O) or a carbonate group (i.e., O-C(=O)-O), preferably a sulfate group, thereby forming the antiviral compound (A) of general formula (I) or the pharmaceutically acceptable salt thereof.

X is preferably selected from fluorine, bromine, chlorine, iodine, OTos or OTMS; more preferably X is fluorine, bromine, chlorine, or iodine, most preferably X is iodine.

Preferably, R¹ is selected from an alkyl group having 1 to 4 carbon atoms, a cyclohexyl, a phenyl or a benzyl and X is fluorine, bromine, chlorine, or iodine. More preferably, R¹ is selected from methyl, ethyl, propyl or isopropyl and X is iodine.

In another preferred aspect, R¹ is selected from an alkyl group having 1 to 4 carbon atoms, a cyclohexyl, a phenyl or a benzyl and Y is a sulfate group (i.e., O-S(=O)₂-O) or a carbonate group (i.e., O-C(=O)-O).

In another particularly preferred aspect, R¹ is selected from methyl, ethyl, propyl or isopropyl and Y is sulfate.

In the present invention, the pharmaceutically acceptable salt of 7-mercapto-3-nitro-1,2,4-triazolo[5,1-c]-1,2,4-triazin-4(1H)-one may be a pharmaceutically acceptable monosalt, a pharmaceutically acceptable disalt or mixtures thereof.

As used herein, the disalt form of 7-mercapto-3-nitro-1,2,4-triazolo[5,1-c]-1,2,4-triazin-4(1H)-one refers to the di-anionic form of 7-mercapto-3-nitro-1,2,4-triazolo[5,1-c]-1,2,4-triazin-4(1H)-one combined with two pharmaceutically acceptable cations, each independently from each other derived from pharmaceutically acceptable non-toxic inorganic or bases organic bases, as detailed above.

As used herein, the monosalt form of 7-mercapto-3-nitro-1,2,4-triazolo[5,1-c]-1,2,4-triazin-4(1H)-one refers to the mono-anionic form of 7-mercapto-3-nitro-1,2,4-triazolo[5,1-c]-1,2,4-triazin-4(1H)-one combined with one cation derived from pharmaceutically acceptable non-toxic inorganic or bases organic bases, as detailed above.

Pharmaceutically acceptable non-toxic inorganic cations notably include sodium, potassium, lithium, ammonia, calcium, magnesium, ferrous, zinc, manganous, aluminum, ferric, manganic cations and the like, sodium cation is especially preferred. Pharmaceutically acceptable organic non-toxic cations notably include cations of primary, secondary, tertiary and quaternary amines, substituted amines including naturally occurring substituted amines, cyclic amines and basic ion exchange resins, such as triethylamine, tripropylamine, 2- dimethylaminoethanol, 2-diethylaminoethanol, lysine, arginine, histidine, caffeine, procaine, N-ethylpiperidine, hydrabamine, choline, betaine, ethylenediamine, glucosamine, methylglycamine, theobromine, purines, piperazine, piperidine, polyamine resins and the like, arginine cation and lysine cation are especially preferred.

In a preferred embodiment of the present invention, 7-mercapto-3-nitro-1,2,4-triazolo[5,1-c]-1,2,4-triazin-4(1H)-one is in the form of the disalt, as defined above.

Generally, the alkylation of 7-mercapto-3-nitro-1,2,4-triazolo[5,1-c]-1,2,4-triazin-4(1 H)-one or a monosalt or a disalt thereof, is generally carried out at ambient temperature.

The Inventors have now surprisingly found that the process for the manufacture of the antiviral compound (A) of general formula (I) or a pharmaceutically acceptable salt thereof, comprising the three steps of diazotization of 5-amino-3-mercapto-1,2,4-triazole (i.e. a compound (B) of general formula (II) having R² being a hydrogen), thereby forming 3-mercapto-1,2,4-triazol-5-yl-diazonium or a monosalt thereof, as detailed above (i.e. Step 1.), followed by condensation reaction of the 3-mercapto-1,2,4-triazol-5-yl-diazonium or the monosalt thereof with at least one α-nitroester of general formula II, as detailed above (i.e. Step 2.), subsequently followed by the alkylation of 7-mercapto-3-nitro-1,2,4-triazolo[5,1-c]-1,2,4-triazin-4(1H)-one or a monosalt or a disalt thereof, as detailed above, can be carried out without isolation of any the intermediates formed in this process according to the invention. This being said, this single-stage process according to the invention allows for improved yield, high efficiency and lower manufacturing cost in particular compared to the known multi-stage processes as notably described by V. L. Rusinov et al. in Pharmaceutical Chemistry Journal, September 1990, Volume 24, Issue 9, pp 646-650, and Russian Pat. Nos. 2294936 and 2536874, as mentioned above.

When R² is equal to R¹, as defined here above, then the antiviral compound (A) of general formula (I) or a pharmaceutically acceptable salt is formed in Step 2. of the process thereby allowing to avoid the final step of alkylation.

There is also a further need that the compound (B) of general formula (II_{R1}), wherein R² is equal to R¹, as shown below, can be easily synthesized in high yield and in a cost effective manner.

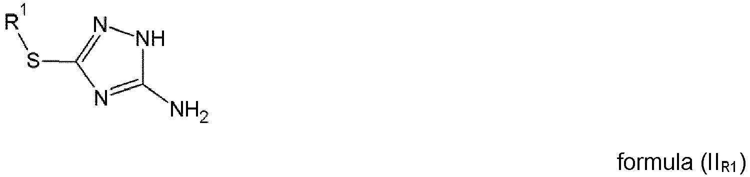

The inventors have now surprisingly found that it is possible to provide an improved process for the manufacture of the compound (B) of general formula (II_{R1}) fulfilling the above mentioned needs.

The process for the manufacture of the compound (B) of general formula (II_{R1}), comprising a condensation reaction of aminoguanidine with a thio compound of general formula (VII): 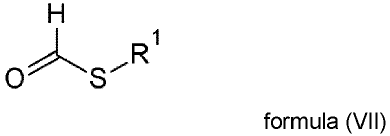 wherein R¹ is selected from an alkyl group having 1 to 12 carbon atoms which is optionally substituted by a halogen atom or hydroxyl group, a cycloalkyl group having 3 to 12 carbon atoms which is optionally substituted by a halogen atom or hydroxyl group, an aryl group, an alkylaryl group, or a cycloheteroalkyl group having 3 to 12 carbon atoms is not forming part of the present invention.

The definitions and preferences described above for the process for the manufacture of the antiviral compound (A) of general formula (I) or a pharmaceutically acceptable salt thereof according to the invention equally apply to the process for the manufacture of the starting material, namely the compound (B) of general formula (II_{R1}).

Preferred thio compounds of general formula (VII) are chosen among S-methyl thio-formate, S-ethyl thioformate, S-propyl thioformate, S-isopropyl thioformate, S-butyl thioformate, S-isobutyl thioformate, S-sec-butyl thioformate, S-tert-butyl thioformate, S-benzyl thioformate, S-phenyl thioformate, S-cyclopropyl thioformate, S-cyclobutyl thioformate, S-cyclopentyl thioformate, S-cyclohexyl thioformate, S-methyl thio-formate is especially preferred.

The condensation reaction of aminoguanidine with the thio compound of general formula (VII), in particular methylthio-formate, may advantageously be carried out in the presence of a base. If a base is used, it may be an inorganic base or an organic base, preferably an organic base. The organic base may be selected from the group consisting of nitrogen-containing heterocyclic compounds such as pyridine, quinoline or picoline; and tertiary bases such as triethylamine, dimethylaniline, diethylaniline and 4-dimethylaminopyridine. Among them, pyridine, triethylamine, dimethylaniline, diethylaniline and 4-dimethylaminopyridine are preferred. Among them, pyridine is particularly preferred. These bases may be used alone or in combination as a mixture.

The inorganic base may be selected from the group consisting of alkali metal hydroxides such as sodium hydroxide, potassium hydroxide, lithium hydroxide, cesium hydroxide, alkaline earth metal hydroxides such as calcium hydroxide, barium hydroxide, magnesium hydroxide, strontium hydroxide, and basic alkali metal salts such as sodium carbonate, sodium hydrogencarbonate, potassium carbonate and potassium hydrogencarbonate. The preferred base is sodium hydroxide.

### EXAMPLES:

The invention will be now described in more details with reference to the following examples, whose purpose is merely illustrative and not intended to limit the scope of the invention.

### Example 1: One pot synthesis of the sodium salt of 7-methylthio-3-nitro [1, 2, 4] triazolo [5,1-c] [1, 2, 4] triazin -4 (1H)-one

**Step 1: Diazotization of compound (B):** A solution (solution [1], herein after) was prepared of 5.8 g (0.05 M) of 5-amino-3-mercapto-1,2,4-triazole in 6.7 ml of nitric acid (15 M) and 12 ml of water. Said solution [1] was refrigerated to -7°C. Then a 40% sodium nitrite solution was added to the solution [1] in portions of 0.5 mL to obtain a total amount of sodium nitrite equal to 3.8 g in the mixture.

**Step 2: Condensation of diazonium compound with an α-nitroester:** To the resulting diazonium salt of step 1, 8.54 ml of diethyl nitromalonate was added. After holding for five minutes, a cooled solution of sodium hydroxide was slowly added dropwise to the reaction mixture until the pH was between pH 9 and pH 10 (solution [2], herein after). The resulting solution [2] was stirred at 0°C for 1 hour and at room temperature for 2 hours.

**Step 3: alkylation:** To the solution [2] of step 2, 6.23 ml (0.1 mol) of methyl iodide was added. The mixture was stirred for 1 hour at room temperature and filtered. The resulting precipitate was successively crystallized from water and dried in air. The reaction scheme is depicted below in Scheme 1.

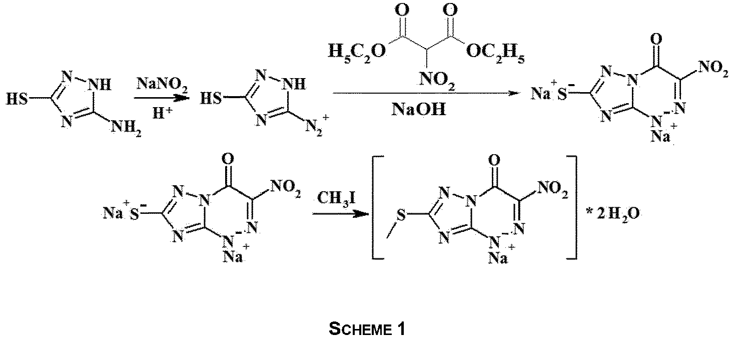

The yield was 9.87 g (69%).

Physical and chemical characteristics of the sodium salt of **7-methylthio-3-nitro [1, 2, 4] triazolo [5,1-c] [1, 2, 4] triazin -4 (1H)-one sodium salt:** yellow crystalline powder, soluble in water, acetone, dimethylsulfoxide, dimethylformamide, insoluble in chloroform; Tₘₑₗₜ = 300°C, ¹H NMR spectrum, δ, ppm, solvent DMSO-d₆: 2.62 (3H, s, SCH₃); IR spectrum, n, cm⁻¹: 3535 (OH), 1649 (C=O), 1505 (NO₂), 1367 (NO₂); found, %: C - 20.86, H 2.51, N 29.28; C₅H₇N₆NaO₅S; Calculated, %: C - 20.98, H 2.47, N 29.36.

### Example 2: Synthesis of the sodium salt of 7-methylthio-3-nitro [1, 2, 4] triazolo [5,1-c] [1, 2, 4] triazin -4 (1H)-one sodium salt

In this example the synthesis comprises 3 steps: in the first step 5-amino-3-mercapto-1,2,4-triazole (i.e. compound (B)) was prepared by condensation of aminoguanidine with a thio-derivative (thio ester) of formic acid, HC(=O)S-R, wherein -R was: methyl. In the second step 5-amino-3-mercapto-1,2,4-triazole was converted to the corresponding diazonium salt. In the third step this diazonium salt was reacted with an α-nitroester, 2-nitroacetoacetic ester, to form the **7-methylthio-3-nitro [1, 2, 4] triazolo [5,1-c] [1, 2, 4] triazin -4 (1H)-one.** The different steps are explained in more detail below.

**Step 1: Synthesis of compound (B):** In a reaction flask equipped with a stirrer, reflux condenser, under inert gas (nitrogen, argon), 20 g (0.1 mol) of aminoguanidine and 7.6 g (0.1 mol) methylthio-formate was added to 400 ml of absolute pyridine. The reaction mixture was boiled for 4 hours at 115°C. Subsequently the reaction mixture was transferred into distilled water and washed several times with water. The washed mixture was dried over a Nutsche filter under vacuum. Recrystallization was carried out from ethanol. The reaction scheme is depicted below in Scheme 2.

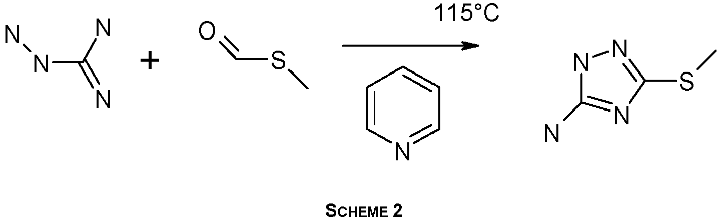

The yield was 19.3 g (70%)

**Step 2: Diazotation of compound (B):** A solution (solution [3], herein after) was prepared of 26 g (0.1 mol) of 5-amino-3-mercapto-1,2,4-triazole (as obtained in step 1) in 32 ml of nitric acid (0.1 mol) and 200 ml of water. The solution was mixed and cooled to -5°C. In a separate recipient, a 0.1 M solution of sodium nitrite was prepared by dissolving 16 g of sodium nitrite in 100 ml of water. The sodium nitrite solution was put in the freezer until there was ice formation and subsequently the ice was crushed. Thereafter, the solution [3] and the sodium nitrite crushed ice were transferred into a 1L reactor and stirred for 1 hour while the reactor temperature was kept at 0°C. The low temperature and the fact that the two reaction components are in different phases (i.e. liquid and solid) ensured a slow gradual progress of diazotization reaction at the phase interface. The end of the diazotization process was controlled by a iodine starch test (proof of the absence of sodium nitrite in a free state).

The reaction scheme is depicted in Scheme 3.

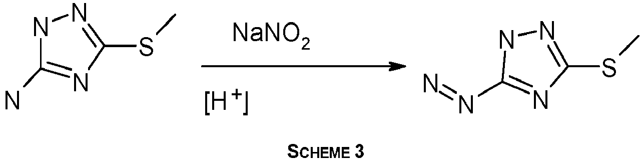

**Step 3: Condensation of the diazonium compound with an α-nitroester:** A solution (solution [4], herein after) was prepared by mixing 17.5 g of methyl 2- nitro-acetoacetate in 300 mL of isopropanol. The solution [4] was mixed with the diazonium salt of step 2. The mixture was cooled to 0°C. At 0°C, a 10% sodium hydroxide solution was added to the reaction mixture (to neutralize residual nitrite and acetate) until there was a marked alkaline reaction (pH between 8 and 9). The temperature was controlled and was kept below +5°C. The resulting mixture was stirred for 1 hour. The precipitate was filtered off and dried in air. The yield was 78%.

The reaction scheme is depicted in Scheme 4

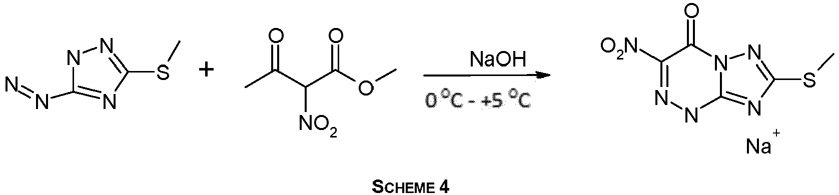

### Example 3: Synthesis of the sodium salt of 7-methylthio-3-nitro [1, 2, 4] triazolo [5,1-c] [1, 2, 4] triazin -4 (1H)-one

The synthesis of the sodium salt of 7-methylthio-3-nitro-1,2,4-triazolo [5,1-c]-1,2,4-triazin-7-one may be carried out as in Example 2, only in step 2 the aqueous alcohol solution is replaced by an alcohol with alkali (such as sodium hydroxide). The yield of the antiviral compound (A) (sodium salt of 7-methylthio-3-nitro [1 2, 4] triazolo [5,1-c] [1, 2, 4] triazin -4 (1H)-one) may increase to 83%.

The reaction scheme is depicted below in Scheme 5:

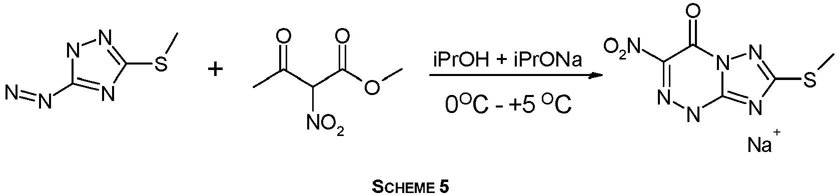

## Claims

1. A process for the manufacture of an antiviral compound (A) of general formula (I) or a pharmaceutically acceptable salt thereof 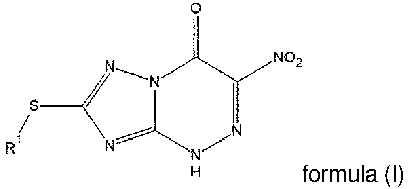 wherein
- R¹ is selected from an alkyl group having 1 to 12 carbon atoms which is optionally substituted by a halogen atom or a hydroxyl group, a cycloalkyl group having 3 to 12 carbon atoms which is optionally substituted by a halogen atom or a hydroxyl group, an aryl group, an alkylaryl group, or a cycloheteroalkyl group having 3 to 12 carbon atoms, preferably an alkyl group having 1 to 8 carbon atoms optionally substituted by a halogen atom or hydroxyl group, a cycloalkyl group having 3 to 6 carbon atoms which is optionally substituted by a halogen atom or hydroxyl group, a phenyl, a benzyl, a morpholine, or an imidazolyl, more preferably an alkyl group having 1 to 4 carbon atoms, a cyclohexyl, a phenyl or a benzyl, and
which comprises the steps of :
Step 1. : diazotization of a compound (B) of general formula (II) : 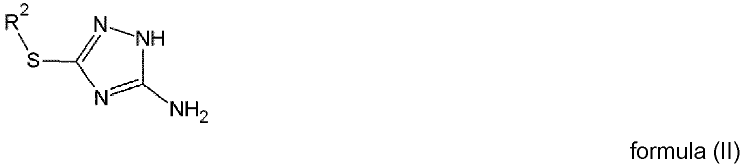 wherein R² is selected from hydrogen, thereby forming a diazonium compound of general formula (III) or a pharmaceutically acceptable salt thereof: 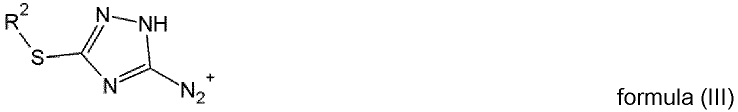 wherein R² has the same meaning as defined here above,
Step 2. : condensation reaction of the diazonium compound of general formula (III) or a pharmaceutically acceptable salt thereof, as obtained in Step 1, with at least one α-nitroester of general formula (IV), as shown below, 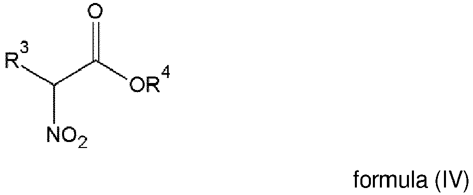 wherein
- R³ is independently selected from a group consisting of:
• -(CO)-OR⁵ wherein R⁵ is an alkyl group having 1 to 24 carbon atoms which is optionally substituted by a hydroxyl group, an amine group, a halogen atom, an aryl group or an aralkyl group;
• -(CO)-R⁶ wherein R⁶ is an alkyl group having 1 to 12 carbon atoms which is optionally substituted by a hydroxyl group, an amine group, a halogen atom, an aryl group or an aralkyl group,
- R⁴ is an alkyl group having 1 to 24 carbon atoms which is optionally substituted by a hydroxyl group, an amine group, a halogen atom, an aryl group or an aralkyl group; an alkenyl group.

2. The process according to claim 1, wherein R¹ is selected from methyl, ethyl, propyl or isopropyl.

3. The process according to claim 1 or claim 2, wherein R¹ is a methyl group.

4. The process according to any one of claims 1 to 3, wherein R³ is -(CO)-OR⁵ wherein R⁵ is an alkyl group having 1 to 24 carbon atoms which is optionally substituted by a hydroxyl group, an amine group, a halogen atom, an aryl group or an aralkyl group.

5. The process according to claim 4, wherein R⁵ is an alkyl group having 1 to 6 carbon atoms.

6. The process according to claim 1 wherein, 7-mercapto-3-nitro-1,2,4-triazolo[5,1-c]-1,2,4-triazin-4(1H)-one or the pharmaceutically acceptable salt thereof, is formed in Step 2. and is further subjected to an alkylation reaction with an alkylating agent of formula (V): R¹-X or formula (VI) R¹-Z wherein R¹ has the same meaning as defined here above, X is a halogen atom or an oxygen containing functional group such as OTos and OTMS, and Z is a sulfate group (i.e., O-S(=O)₂-O) or a carbonate group (i.e., O-C(=O)-O), preferably a sulfate group.

7. A process for the manufacture of an antiviral compound (A) of general formula (I) or a pharmaceutically acceptable salt thereof 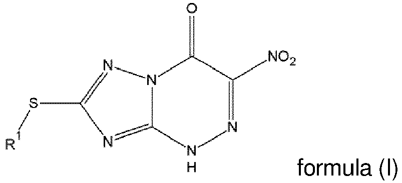 wherein
- R¹ is selected from an alkyl group having 1 to 12 carbon atoms which is optionally substituted by a halogen atom or hydroxyl group, a cycloalkyl group having 3 to 12 carbon atoms which is optionally substituted by a halogen atom or hydroxyl group, an aryl group, an alkylaryl group, or a cycloheteroalkyl group having 3 to 12 carbon atoms, and
which comprises the steps of :
Step 1. : diazotization of a compound (B) of general formula (II) : 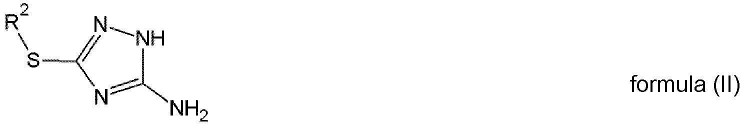 wherein R² is equal to R¹, as defined here above, thereby forming a diazonium compound of general formula (III) or a pharmaceutically acceptable salt thereof: 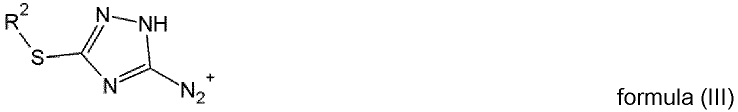 wherein R² has the same meaning as defined here above,
Step 2. : condensation reaction of the diazonium compound of general formula (III) or a pharmaceutically acceptable salt thereof, as obtained in Step 1, with at least one α-nitroester of general formula (IV), as shown below, 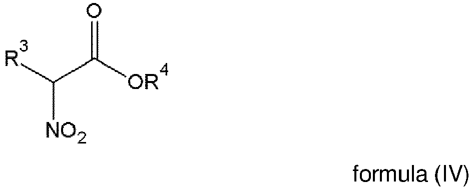 wherein
- R³ is -(CO)-R⁶ wherein R⁶ is an alkyl group having 1 to 12 carbon atoms which is optionally substituted by a hydroxyl group, an amine group, a halogen atom, an aryl group or an aralkyl group,
- R⁴ is an alkyl group having 1 to 24 carbon atoms which is optionally substituted by a hydroxyl group, an amine group, a halogen atom, an aryl group or an aralkyl group; an alkenyl group.

8. The process according to claim 7, wherein R⁶ is an alkyl group having 1 to 6 carbon atoms, preferably an alkyl group having 1 to 4 carbon atoms.

9. The process according to any one of claims 1 to 8, wherein the molar ratio of the diazonium compound of general formula (III) to the α-nitroester of general formula (IV) is from 0.5:2 to 2:0.5, preferably from 0.9:1 to 1:0.9.

10. The process according to any one of claims 1 to 9, wherein the condensation reaction in Step 2. is carried out in the presence of at least one pharmaceutically acceptable non-toxic inorganic or organic base.

11. The process according to any one of claims 1 to 10, wherein the α-nitroester of general formula (IV) is used in combination with a solvent selected from an alcohol such as methanol, ethanol, n-propyl alcohol, isopropyl alcohol, tert-butyl alcohol, n-butyl alcohol, iso-butyl alcohol and the like; an ether such as dimethylether, diethylether, dipropylether, diisopropylether, di-tert-butylether, diisobutylether, dibutylether, di-sec-butylether, dioxane, tetrahydrofuran, dimethoxymethane, dimethoxyethane, dimethoxypropane; a ketone such as dimethylketone, diethylketone, dipropylketone, methylethylketone; a sulfur-containing solvent such as dimethylsulfoxide, dimethylsulfone, diphenylsulfone, diethylsulfoxide, diethylsulfone, diisopropylsulfone, tetrahydrothiophene-1, 1-dioxide (commonly called tetramethylene sulfone or sulfolane), tetrahydrothiophene-1-monoxide; a nitrogen-containing polar aprotic solvent such as dimethylacetamide, dimethylformamide and N-methyl pyrrolidinone (i.e., NMP); and mixtures thereof.

## Patentansprüche

1. Ein Verfahren zur Herstellung einer antiviralen Verbindung (A) der allgemeinen Formel (I) oder eines pharmazeutisch akzeptablen Salzes davon, 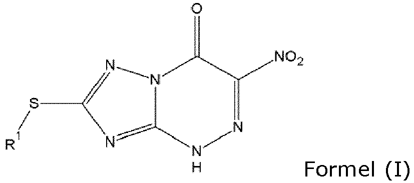 wobei
- R¹ ausgewählt ist aus einer Alkylgruppe mit 1 bis 12 Kohlenstoffatomen, welche gegebenenfalls substituiert ist durch ein Halogenatom oder eine Hydroxylgruppe, einer Cycloalkylgruppe mit 3 bis 12 Kohlenstoffatomen, welche gegebenenfalls substituiert ist durch ein Halogenatom oder eine Hydroxylgruppe, einer Arylgruppe, einer Alkylarylgruppe, oder einer Cycloheteroalkylgruppe mit 3 bis 12 Kohlenstoffatomen, bevorzugt einer Alkylgruppe mit 1 bis 8 Kohlenstoffatomen gegebenenfalls substituiert durch ein Halogenatom oder eine Hydroxylgruppe, einer Cycloalkylgruppe mit 3 bis 6 Kohlenstoffatomen, welche gegebenenfalls substituiert ist durch ein Halogenatom oder eine Hydroxylgruppe, einem Phenyl, einem Benzyl, einem Morpholin, oder einem Imidazolyl, mehr bevorzugt einer Alkylgruppe mit 1 bis 4 Kohlenstoffatomen, einem Cyclohexyl, einem Phenyl oder einem Benzyl, und
welches folgende Schritte umfasst:
Schritt 1.: Diazotierung einer Verbindung (B) der allgemeinen Formel (II): 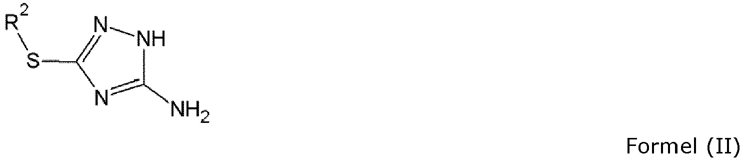 wobei R² ausgewählt ist aus Wasserstoff, wodurch eine Diazoniumverbindung der allgemeinen Formel (III) oder ein pharmazeutisch akzeptables Salz davon gebildet wird: 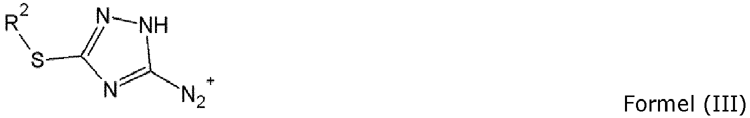 wobei R² dieselbe Bedeutung hat, wie hier oben definiert,
Schritt 2.: Kondensationsreaktion der Diazoniumverbindung der allgemeinen Formel (III) oder eines pharmazeutisch akzeptablen Salzes davon, wie erhalten in Schritt 1., mit zumindest einem α-Nitroester der allgemeinen Formel (IV), wie unten dargestellt, 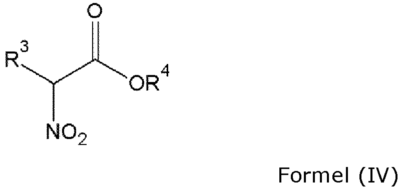 wobei
- R³ unabhängig ausgewählt ist aus einer Gruppe bestehend aus:
• -(CO)-OR⁵, wobei R⁵ eine Alkylgruppe mit 1 bis 24 Kohlenstoffatomen ist, welche gegebenenfalls substituiert ist durch eine Hydroxylgruppe, eine Amingruppe, ein Halogenatom, eine Arylgruppe oder eine Aralkylgruppe;
• -(CO)-OR⁶, wobei R⁶ eine Alkylgruppe mit 1 bis 12 Kohlenstoffatomen ist, welche gegebenenfalls substituiert ist durch eine Hydroxylgruppe, eine Amingruppe, ein Halogenatom, eine Arylgruppe oder eine Aralkylgruppe,
- R⁴ eine Alkylgruppe mit 1 bis 24 Kohlenstoffatomen ist, welche gegebenenfalls substituiert ist durch eine Hydroxylgruppe, eine Amingruppe, ein Halogenatom, eine Arylgruppe oder eine Aralkylgruppe; eine Alkenylgruppe.

2. Das Verfahren nach Anspruch 1, wobei R¹ ausgewählt ist aus Methyl, Ethyl, Propyl oder Isopropyl.

3. Das Verfahren nach Anspruch 1 oder Anspruch 2, wobei R¹ eine Methylgruppe ist.

4. Das Verfahren nach irgendeinem der Ansprüche 1 bis 3, wobei R³ -(CO)-OR⁵ ist, wobei R⁵ eine Alkylgruppe mit 1 bis 24 Kohlenstoffatomen ist, welche gegebenenfalls substituiert ist durch eine Hydroxylgruppe, eine Amingruppe, ein Halogenatom, eine Arylgruppe oder eine Aralkylgruppe.

5. Das Verfahren nach Anspruch 4, wobei R⁵ eine Alkylgruppe mit 1 bis 6 Kohlenstoffatomen ist.

6. Das Verfahren nach Anspruch 1, wobei 7-Mercapto-3-nitro-1,2,4-triazolo[5,1-c]-1,2,4-triazin-4(1H)-on oder das pharmazeutisch akzeptable Salz davon, in Schritt 2. gebildet wird und weiter einer Alkylierungsreaktion mit einem Alkylans der folgenden Formel (V) unterzogen wird: R¹-X oder Formel (VI) R¹-Z, wobei R¹ dieselbe Bedeutung hat wie hier oben definiert, X ein Halogenatom oder eine sauerstoffhaltige funktionelle Gruppe wie OTos und OTMS ist, und Z eine Sulfatgruppe (d. h., O-S(=O)₂-O) oder eine Carbonatgruppe (d. h., O-C(=O)-O), bevorzugt eine Sulfatgruppe ist.

7. Ein Verfahren zur Herstellung einer antiviralen Verbindung (A) der allgemeinen Formel (I) oder eines pharmazeutisch akzeptablen Salzes davon, 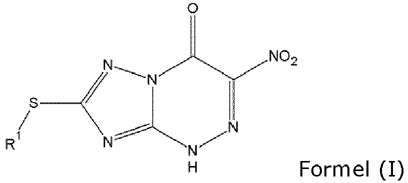 wobei
- R¹ ausgewählt ist aus einer Arylgruppe mit 1 bis 12 Kohlenstoffatomen, welche gegebenenfalls substituiert ist durch ein Halogenatom oder eine Hydroxylgruppe, einer Cycloalkylgruppe mit 3 bis 12 Kohlenstoffatomen, welche gegebenenfalls substituiert ist durch ein Halogenatom oder eine Hydroxylgruppe, einer Arylgruppe, einer Alkylarylgruppe, oder einer Cycloheteroalkylgruppe mit 3 bis 12 Kohlenstoffatomen, und
welches folgende Schritte umfasst:
Schritt 1.: Diazotierung einer Verbindung (B) der allgemeinen Formel (II): 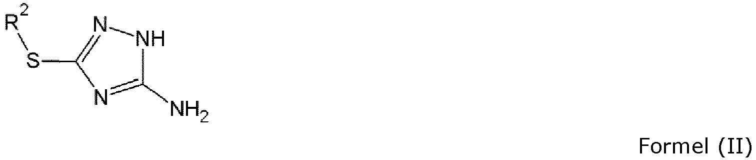 wobei R² gleich R¹ ist, wie hier oben definiert, wodurch eine Diazoniumverbindung der allgemeinen Formel (III) oder ein pharmazeutisch akzeptables Salz davon gebildet wird: 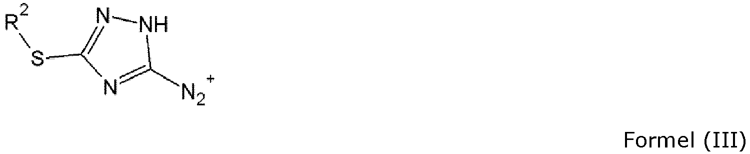 wobei R² dieselbe Bedeutung hat, wie hier oben definiert,
Schritt 2.: Kondensationsreaktion der Diazoniumverbindung der allgemeinen Formel (III) oder eines pharmazeutisch akzeptablen Salzes davon, wie erhalten in Schritt 1., mit zumindest einem α-Nitroester der allgemeinen Formel (IV), wie unten dargestellt, 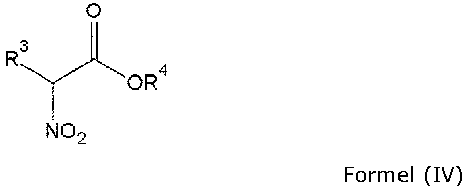 wobei
- R³ -(CO)-OR⁶ ist, wobei R⁶ eine Alkylgruppe mit 1 bis 12 Kohlenstoffatomen ist, welche gegebenenfalls substituiert ist durch eine Hydroxylgruppe, eine Amingruppe, ein Halogenatom, eine Arylgruppe oder eine Aralkylgruppe,
- R⁴ eine Alkylgruppe mit 1 bis 24 Kohlenstoffatomen ist, welche gegebenenfalls substituiert ist durch eine Hydroxylgruppe, eine Amingruppe, ein Halogenatom, eine Arylgruppe oder eine Aralkylgruppe; eine Alkenylgruppe.

8. Das Verfahren nach Anspruch 7, wobei R⁶ eine Alkylgruppe mit 1 bis 6 Kohlenstoffatomen ist, bevorzugt eine Alkylgruppe mit 1 bis 4 Kohlenstoffatomen.

9. Das Verfahren nach irgendeinem der Ansprüche 1 bis 8, wobei das Molverhältnis von der Diazoniumverbindung der allgemeinen Formel (III) zum α-Nitroester der allgemeinen Formel (IV) von 0,5:2 bis 2:0,5, bevorzugt von 0,9:1 bis 1:0,9 beträgt.

10. Das Verfahren nach irgendeinem der Ansprüche 1 bis 9, wobei die Kondensationsreaktion in Schritt 2. in Anwesenheit von zumindest einer pharmazeutisch akzeptablen nicht toxischen anorganischen oder organischen Base ausgeführt wird.

11. Das Verfahren nach irgendeinem der Ansprüche 1 bis 10, wobei der α-Nitroester der allgemeinen Formel (IV) verwendet wird in Kombination mit einem Lösungsmittel, ausgewählt aus einem Alkohol wie Methanol, Ethanol, n-Propylalkohol, Isopropylalkohol, tert-Butylalkohol, n-Butylalkohol, Isobutylalkohol und Ähnlichem; einem Ether wie Dimethylether, Diethylether, Dipropylether, Diisopropylether, Di-tert-Butylether, Diisobutylether, Dibutylether, Di-sec-Butylether, Dioxan, Tetrahydrofuran, Dimethoxymethan, Dimethoxyethan, Dimethoxypropan; einem Keton wie Dimethylketon, Diethylketon, Dipropylketon, Methylethylketon; einem schwefelhaltigen Lösungsmittel wie Dimethylsulfoxid, Dimethylsulfon, Diphenylsulfon, Diethylsulfoxid, Diethylsulfon, Diisopropylsulfon, Tetrahydrothiophen-1, 1-dioxid (allgemein Tetramethylensulfon oder Sulfolan genannt), Tetrahydrothiophen-1-monoxid; einem stickstoffhaltigen polaren aprotischen Lösungsmittel wie Dimethylacetamid, Dimethylformamid und N-Methylpyrrolidinon (d. h., NMP); und Mischungen davon.

## Revendications

1. Procédé pour la fabrication d'un composé antiviral (A) de formule générale (I) ou d'un sel pharmaceutiquement acceptable de celui-ci 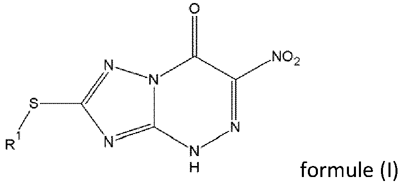 dans lequel
- R¹ est choisi parmi un groupe alkyle ayant 1 à 12 atomes de carbone qui est éventuellement substitué par un atome d'halogène ou un groupe hydroxyle, un groupe cycloalkyle ayant 3 à 12 atomes de carbone qui est éventuellement substitué par un atome d'halogène ou un groupe hydroxyle, un groupe aryle, un groupe alkylaryle, ou un groupe cyclohétéroalkyle ayant 3 à 12 atomes de carbone, de préférence un groupe alkyle ayant 1 à 8 atomes de carbone éventuellement substitué par un atome d'halogène ou un groupe hydroxyle, un groupe cycloalkyle ayant 3 à 6 atomes de carbone qui est éventuellement substitué par un atome d'halogène ou un groupe hydroxyle, un groupe phényle, un groupe benzyle, un groupe morpholine ou un groupe imidazolyle, plus préférablement un groupe alkyle ayant 1 à 4 atomes de carbone, un groupe cyclohexyle, un groupe phényle ou un groupe benzyle, et
qui comprend les étapes suivantes :
Étape 1 : diazotation d'un composé (B) de formule générale (II) : 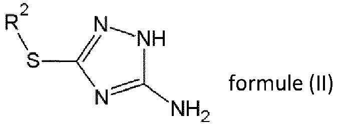 dans lequel R² est choisi parmi l'hydrogène, formant ainsi un composé diazonium de formule générale (III) ou un sel pharmaceutiquement acceptable de celui-ci : 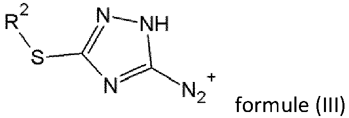 dans lequel R² a la même signification que celle définie ci-dessus,
Étape 2 : réaction de condensation du composé diazonium de formule générale (III) ou d'un sel pharmaceutiquement acceptable de celui-ci, tel qu'obtenu à l'Étape 1, avec au moins un α-nitroester de formule générale (IV), comme indiqué ci-dessous, 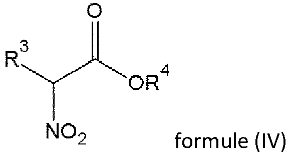 dans lequel
- R³ est choisi indépendamment dans un groupe constitué de :
• -(CO)-OR⁵ où R⁵ représente un groupe alkyle ayant 1 à 24 atomes de carbone qui est éventuellement substitué par un groupe hydroxyle, un groupe amine, un atome d'halogène, un groupe aryle ou un groupe aralkyle ;
• -(CO)-R⁶ où R⁶ représente un groupe alkyle ayant 1 à 12 atomes de carbone qui est éventuellement substitué par un groupe hydroxyle, un groupe amine, un atome d'halogène, un groupe aryle ou un groupe aralkyle,
- R⁴ représente un groupe alkyle ayant 1 à 24 atomes de carbone qui est éventuellement substitué par un groupe hydroxyle, un groupe amine, un atome d'halogène, un groupe aryle ou un groupe aralkyle ; un groupe alcényle.

2. Procédé selon la revendication 1, dans lequel R¹ est choisi parmi méthyle, éthyle, propyle ou isopropyle.

3. Procédé selon la revendication 1 ou la revendication 2, dans lequel R¹ représente un groupe méthyle.

4. Procédé selon l'une quelconque des revendications 1 à 3, dans lequel R³ représente -(CO)-OR⁵ dans lequel R⁵ représente un groupe alkyle ayant 1 à 24 atomes de carbone qui est éventuellement substitué par un groupe hydroxyle, un groupe amine, un atome d'halogène, un groupe aryle ou un groupe aralkyle.

5. Procédé selon la revendication 4, dans lequel R⁵ représente un groupe alkyle ayant 1 à 6 atomes de carbone.

6. Procédé selon la revendication 1, dans lequel la 7-mercapto-3-nitro-1,2,4-triazolo[5,1-c]-1,2,4-triazin-4(1H)-one ou un sel pharmaceutiquement acceptable de celui-ci, est formée à l'Étape 2. et est en outre soumise à une réaction d'alkylation avec un agent d'alkylation de formule (V) : R¹-X ou formule (VI) R¹-Z dans lequel R¹ a la même signification que celle définie ci-dessus, X représente un atome d'halogène ou un groupe fonctionnel contenant de l'oxygène tel que OTos et OTMS, et Z représente un groupe sulfate (c-à-d., O-S(=O)₂-O) ou un groupe carbonate (c-à-d., O-C(=O)-O), de préférence un groupe sulfate.

7. Procédé pour la fabrication d'un composé antiviral (A) de formule générale (I) ou d'un sel pharmaceutiquement acceptable de celui-ci 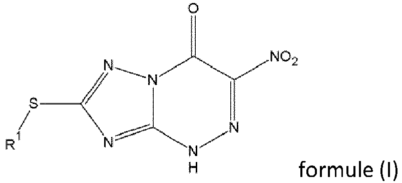 dans lequel
- R¹ est choisi parmi un groupe alkyle ayant 1 à 12 atomes de carbone qui est éventuellement substitué par un atome d'halogène ou un groupe hydroxyle, un groupe cycloalkyle ayant 3 à 12 atomes de carbone qui est éventuellement substitué par un atome d'halogène ou un groupe hydroxyle, un groupe aryle, un groupe alkylaryle ou un groupe cyclohétéroalkyle ayant 3 à 12 atomes de carbone, et
qui comprend les étapes suivantes
Étape 1 : diazotation d'un composé (B) de formule générale (II) : 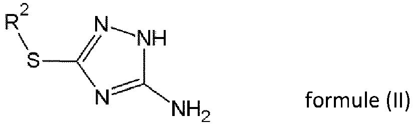 dans lequel R² est égal à R¹, tel que défini ci-dessus, formant ainsi un composé diazonium de formule générale (III) ou un sel pharmaceutiquement acceptable de celui-ci : 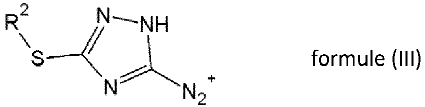 dans lequel R² a la même signification que celle définie ci-dessus,
Étape 2 : réaction de condensation du composé diazonium de formule générale (III) ou d'un sel pharmaceutiquement acceptable de celui-ci, tel qu'obtenu à l'Étape 1, avec au moins un α-nitroester de formule générale (IV), comme indiqué ci-dessous, 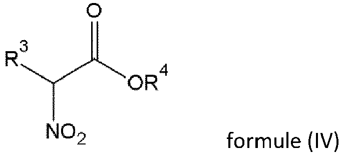 dans lequel
- R³ représente -(CO)-R⁶ où R⁶ représente un groupe alkyle ayant 1 à 12 atomes de carbone qui est éventuellement substitué par un groupe hydroxyle, un groupe amine, un atome d'halogène, un groupe aryle ou un groupe aralkyle,
- R⁴ représente un groupe alkyle ayant 1 à 24 atomes de carbone qui est éventuellement substitué par un groupe hydroxyle, un groupe amine, un atome d'halogène, un groupe aryle ou un groupe aralkyle ; un groupe alcényle.

8. Procédé selon la revendication 7, dans lequel R⁶ représente un groupe alkyle ayant 1 à 6 atomes de carbone, de préférence un groupe alkyle ayant 1 à 4 atomes de carbone.

9. Procédé selon l'une quelconque des revendications 1 à 8, dans lequel le rapport molaire du composé diazonium de formule générale (III) sur l'α-nitroester de formule générale (IV) est compris entre 0,5:2 et 2:0,5, de préférence entre 0,9:1 et 1:0,9.

10. Procédé selon l'une quelconque des revendications 1 à 9, dans lequel la réaction de condensation de l'Étape 2. est réalisée en présence d'au moins une base inorganique ou organique non toxique pharmaceutiquement acceptable.

11. Procédé selon l'une quelconque des revendications 1 à 10, dans lequel l'α-nitroester de formule générale (IV) est utilisé en combinaison avec un solvant choisi parmi un alcool tel que le méthanol, l'éthanol, l'alcool n-propylique, l'alcool isopropylique, l'alcool tert-butylique, l'alcool n-butylique, l'alcool iso-butylique, et similaires ; un éther tel que le diméthyléther, le diéthyléther, le dipropyléther, le diisopropyléther, le di-tert-butyléther, le diisobutyléther, le dibutyléther, le di-sec-butyléther, le dioxane, le tétrahydrofurane, le diméthoxyméthane, le diméthoxyéthane, le diméthoxypropane ; une cétone telle que la diméthylcétone, la diéthylcétone, la dipropylcétone, la méthyléthylcétone ; un solvant contenant du soufre tel que le diméthylsulfoxyde, la diméthylsulfone, la diphénylsulfone, le diéthylsulfoxyde, la diéthylsulfone, la diisopropylsulfone, le tétrahydrothiophène-1, le 1-dioxyde (communément appelé tétraméthylène sulfone ou sulfolane), le tétrahydrothiophène-1-monoxyde ; un solvant aprotique polaire contenant de l'azote, tel que le diméthylacétamide, le diméthylformamide et la N-méthylpyrrolidinone (c-à-d., NMP) ; et leurs mélanges.
